# EUROPEAN PATENT SPECIFICATION

(11) **EP 3 793 445 B1**
(45) Date of publication and mention of the grant of the patent: **10.01.2024**
(21) Application number: 19732466.8
(22) Date of filing: 13.05.2019
(51) Int. Cl.: A61B 7/00

(54) **SYSTEM FOR DIGITIZING THE SOUND OF A STETHOSCOPE AND FOR SENDING IT TO AN ELECTRONIC INSTRUMENT**
SYSTEM ZUR DIGITALISIERUNG DES GERÄUSCHS EINES STETHOSKOPS UND ZUM SENDEN DAVON AN EIN ELEKTRONISCHES INSTRUMENT
SYSTÈME PERMETTANT DE NUMÉRISER LE SON D'UN STÉTHOSCOPE ET DE L'ENVOYER À UN INSTRUMENT ÉLECTRONIQUE

(30) Priority: 14.05.2018 IT 201800005326
(43) Date of publication of application: 24.03.2021
(73) Proprietor: ARCON S.R.L., 88100 CATANZARO (CZ) (IT)
(72) Inventor: BONANNO, Marco, 88100 Catanzaro (CZ) (IT)
(74) Representative: Giuliano, Natalia
(86) International application number: PCT/IB2019/053943
(87) International publication number: WO 2019/220316

(56) References cited:
- WO-A1-03/063707
- WO-A1-2015/094426
- US-A1- 2004 076 303

## Description

The present invention is related to a system for digitizing the sound of a stethoscope and for sending it to an electronic instrument.

As it is known, the acoustic stethoscope, also known as stethoscope or phonendoscope, is an essential device in order to make the most relevant part of the objective medical examination: the auscultation, such as the auscultation of the heart, of lungs, of abdomen, of several arteries, etc. This instrument is also necessary to measure the arterial pressure by means of a manual sphygmomanometer, usually employed by patients or by healthcare professional. The stethoscope is provided at one end with a membrane that detects the sound waves having higher frequencies, sometimes accompanied by a bell that detects the sound waves having lower frequencies; the central part of the stethoscope is a flexible tube through which the sound waves, once detected, are modified and transmitted to the human ear by means of two eartips that are located at the other end of the stethoscope and rest on the outer ear of the listener, as described in the paper "Comparison of the acoustic properties of six popular stethoscopes", by Abella M, Formolo J, Penney DG published on J. Acoust's Am. Soc. Apr. 1992; 91(4 Pt 1):2224-8. In this way the entire stethoscope affects the sound heard by the user, so that even the sound features often depend on the brand and on the model of the phonendoscope, so as to persuade the user to employ only a specific stethoscope model.

Several digital stethoscopes, however rarely employed, are known on the market and these replace the traditional stethoscopes being used to amplify the sounds and/or to send them to electronic devices, i.e. to personal computers. By way of example, there is an electronic stethoscope made by Littmann, a market leader in the field of stethoscopes. This instrument is expensive and produces a type of sound different from the sound to which the healthcare professionals are used to. Being such a device powered by batteries, in addition, it stops functioning if the batteries are out of charge, this representing an issue in case of a medical urgency, so that it is necessary to own and to have at hand also a traditional stethoscope.

A first known solution available in the state of the art is described in the patent application US2004076303 which discloses a multimedia acoustic stethoscope that has look and feel of a conventional acoustic stethoscope but allows a medical practitioner to transmit sounds from the stethoscope to a recording device, and to transmit recorded sound back to the stethoscope for playback. The recording device could be a tape recorder, a digital recorder, a personal computer, a pocket PC, a handheld PC, a tablet PC, or a PDA. The data can be transmitted via a wire or wirelessly, for example using a Bluetooth connection.

Another example is the international application WO2015094426 that describes an input monitoring and recording device for medical diagnostics. Specifically, the invention relates to a device that may be used for monitoring and recording physiological parameters and auscultation sounds that are measured by a diagnostic or an auscultation instrument. The device may be further used to transmit any collected inputs, which relate to the biological parameters of a person's body, for analysis made by a remotely located healthcare professional.

A further example is the utility model application CN202960564 that discloses a modified stethoscope using a wireless connection to send detected data to a visualization device.

Another example is the patent application KR2013022141 which discloses a modified stethoscope connected to a smartphone that includes a microphone and a means to process and to convert the sound wave received by the microphone into an electric signal.

Again, the utility model application CN203138540 discloses a modified smartphone having the features of a stethoscope.

Moreover, the international application WO2006090964 describes a modified smartphone that connects to a modified stethoscope.

Another solution is described in the patent application CN202821420 having as its subject a modified stethoscope with a microphone placed into the chestpiece and a wire inserted into the tube that connects the microphone to a speaker placed into an eartip.

In addition, the international application WO2015094426 describes a device for monitoring input signals and for registering in the field of medical diagnostics, in particular monitoring recorded physiologic parameters and auscultation sounds measured by a diagnostic instrument. In addition, the device transmits and receives input signals concerning biological parameters of the body of a person, to allow distant medical centers to be able to make analysis from afar.

Finally, a modified stethoscope (StethoCloud) capable of connecting to the jacks of smartphones, has been sold for a while.

However, no solution capable of converting the sounds detected by a common stethoscope, without technical changes, into digital data, sending those data to electronic instruments, such as headphones, computers or smartphones, is available on the market; all this in order to use instruments already owned by patients or healthcare professionals, and to listen to the sound familiar to those who use a stethoscope and have specific sound references about various human diseases.

A solution has been disclosed by the same Applicant through the Italian patent application N. UB2015A001919 that describes a device for digitizing the sound of a stethoscope and for sending it to an electronic device, comprising a solid body provided with lateral slots able to house the eartips of the stethoscope in an active measurement configuration, with a hooking lip and with a system to hook the device to the membrane and to the Y-tube of the stethoscope in a passive rest configuration.

However, there are no inventions that digitize the traditional stethoscope without modifying it, keeping inserted the eartips directly into the user's ears for the auscultation in real time. Indeed, even in the previous patent application of the Applicant the user, even using the traditional stethoscope, has to use headphones to listen in real time.

The purpose of the present invention is to provide a system for digitizing the sound of a stethoscope and for sending it to an electronic instrument and that is simple, compact and not expensive having, therefore, features so as to overcome the limitations that still affect the known technical solutions.

Another purpose of the present invention is to provide a system for digitizing the sound of a stethoscope and for sending it to an electronic instrument that is wearable, in use, like a traditional stethoscope.

According to the present invention, a system for digitizing the sound of a stethoscope and for sending it to an electronic instrument is provided, as defined in claim 1.

For a better understanding of the present invention a preferred embodiment is now described, purely by way of non-limiting example, with reference to annexed drawings, in which:
- the figure 1 shows a stethoscope equipped with eartips, a tube and a membrane according to the prior art;
- the figure 2 shows a schematic view of a first embodiment of a system for digitizing the sound of a stethoscope and for sending it to an electronic instrument, according to a non-claimed embodiment.
- the figure 3 shows a schematic view of a second embodiment of a system for digitizing the sound of a stethoscope and for sending it to an electronic instrument, according to a non-claimed embodiment.
- the figure 4 shows a schematic view of a third embodiment of a system for digitizing the sound of a stethoscope and for sending it to an electronic instrument, according to the invention;
- the figure 5 shows a schematic view of a fourth embodiment of a system for digitizing the sound of a stethoscope and for sending it to an electronic instrument, according to the invention;
- the figures 6a and 6b show a schematic view of a known stethoscope and a system for digitizing the sound of a stethoscope applied to a known stethoscope, according to the invention;
- the figures 7a and 7b show a detail view of a transducer-loudspeaker assembly of a system for digitizing the sound of a stethoscope and for sending it to an electronic instrument, applied to one end of the stethoscope, according to the invention.

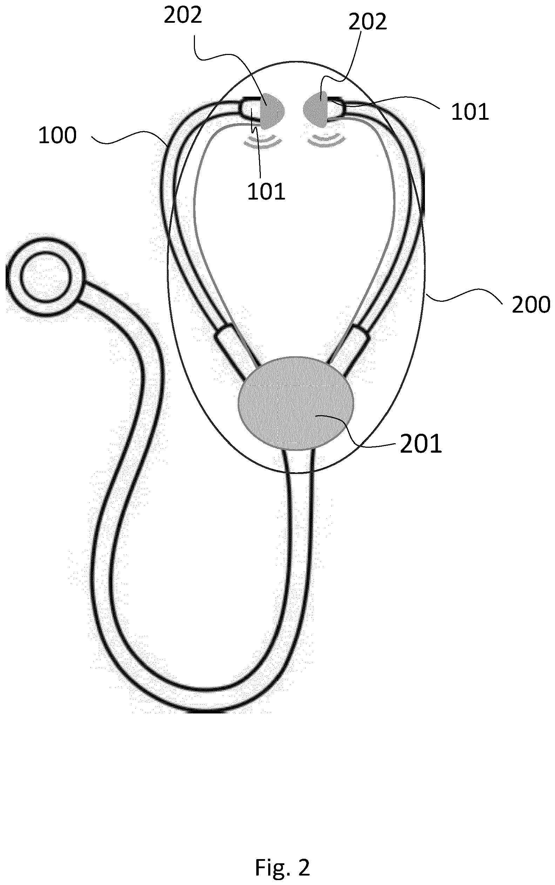

With reference to these figures and, in particular, to figure 2, a first non-claimed embodiment of a system for digitizing the sound of a stethoscope and for sending it to an electronic instrument is shown, according to the invention.

In particular, the system 200 for digitizing the sound of a stethoscope 100 equipped with eartips 101 and a tube, and for sending said sound to an electronic instrument comprises a transducer-loudspeaker assembly 202 composed of a loudspeaker and a transducer, wherein the transducer is configured to digitize the sound coming from the eartips 101, and a body 201 configured to receive the digitized sound from the transducer and to amplify, filter and send said sound to the loudspeaker, said loudspeaker being configured to reproduce the sound processed by the body 201.

The transducer-loudspeaker assembly 202 is placed at one end of the stethoscope 100.

In such a first non-claimed embodiment, the transducer-loudspeaker assembly 202 is hooked to at least an eartip 101 of the traditional stethoscope 100.

The transducer is a microphone capsule.

In use, the transducer-loudspeaker assembly 202 sends the signal through wire, or by a wireless connection (i.e. Bluetooth) to the body 201 having the function of processing the sound; said body 201 acquires the signal digitized by the transducer and is able to amplify, filter, store and send it. Once the signal is processed by the body 201, the signal itself, through wire or by a wireless connection (i.e. a Bluetooth connection) is sent to the loudspeaker of the transducer-loudspeaker assembly 202, said loudspeaker being able to be housed into the user's ears for the auscultation. In addition, the body 201, having the function of processing the sound, sends the signal through wire or by a wireless connection to electronic devices such as smartphones, tablets, computers, ICT platforms.

According to this first non-claimed embodiment, in the system 200 the transducer and the loudspeaker represent a single block placed on the eartip 101 of the stethoscope 100 so that the transducer is able to connect to the eartip 101 and the loudspeaker is placed into the user's ears.

In the first non-claimed embodiment the body 201 having the function of amplifying, filtering and transmitting the sound is hooked to the tube of the stethoscope 100, and the transducer-loudspeaker assembly 202 communicates with the body 201 through wire or by a wireless connection (Bluetooth, Wi-Fi, etc.)

The body 201 has a concavity having a half-moon shape that is able to house the membrane/capsule of the traditional stethoscope 100 when not in use.

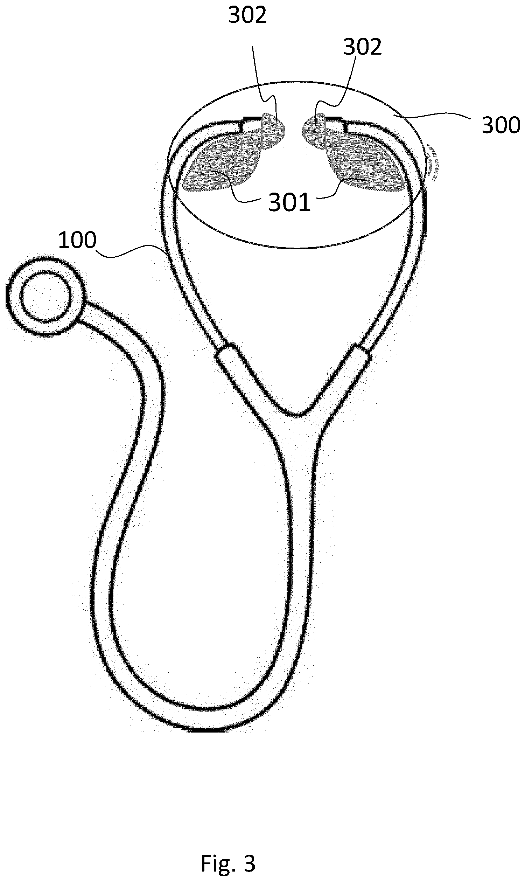

According to a second non-claimed embodiment, shown in figure 3, the system 300 for digitizing the sound of a stethoscope 100, equipped with eartips 101 and a tube, and for sending said sound to an electronic instrument comprises a body 301 having a processing function that is integrated with a transducer-loudspeaker assembly 302 composed by a loudspeaker and a transducer, so that the entire system 300 is a single compact system hooked to the eartips 101 of the traditional stethoscope.

According to this second non-claimed embodiment, also in the system 300 the transducer is configured to digitize the sound coming from the eartips 101, the body 301 is configured to amplify, filter and send said sound to the loudspeaker, and the loudspeaker is configured to reproduce the sound processed by the body 301.

According to this second non-claimed embodiment, also in the system 300 the transducer-loudspeaker assembly 302 is placed at one end of the stethoscope 100.

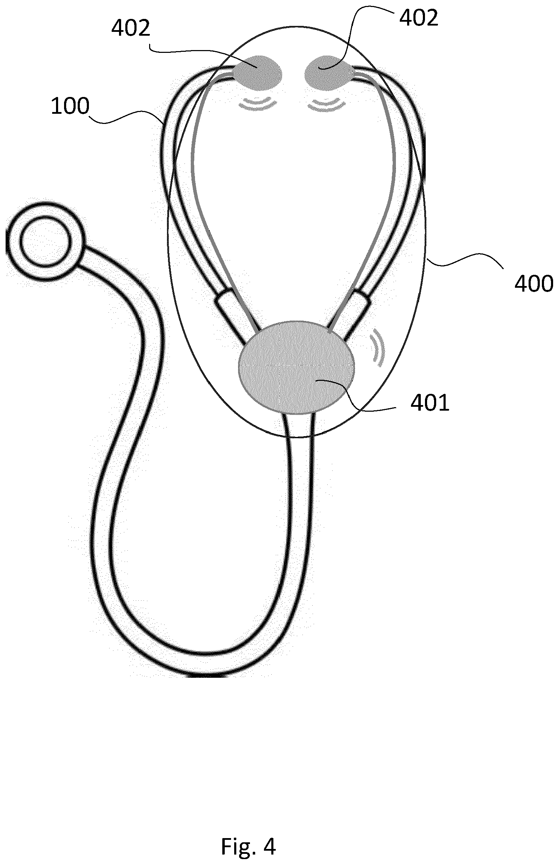

In figure 4 a third embodiment of the system 400 for digitizing the sound of a stethoscope 100, the stethoscope 100 being equipped with eartips 101 and a tube, and for sending said sound to an electronic instrument is shown, said third embodiment comprising a body 401 having a processing function and that is hooked to the tube of the stethoscope 100, and a transducer-loudspeaker assembly 402, composed of a transducer and a loudspeaker, directly connected to the metallic tube of the stethoscope 100 substituting at least one eartip 101 of the traditional stethoscope, so turning itself into a substitute electronic eartip.

According to this third embodiment, also in the system 400 the transducer is configured to digitize the sound coming from the stethoscope 100, the body 401 is configured to receive the digitized sound from the transducer and to amplify, filter and send said sound to the loudspeaker, said loudspeaker being configured to reproduce the sound processed by the body 401.

According to this third embodiment, also in the system 400 the transducer-loudspeaker assembly 402 is placed at one end of the stethoscope 100.

According to this third embodiment, also in the system 400 the body 401 is configured to send the amplified and filtered digital signal via a cable or wireless connection to the loudspeaker and to fixed or portable electronic devices.

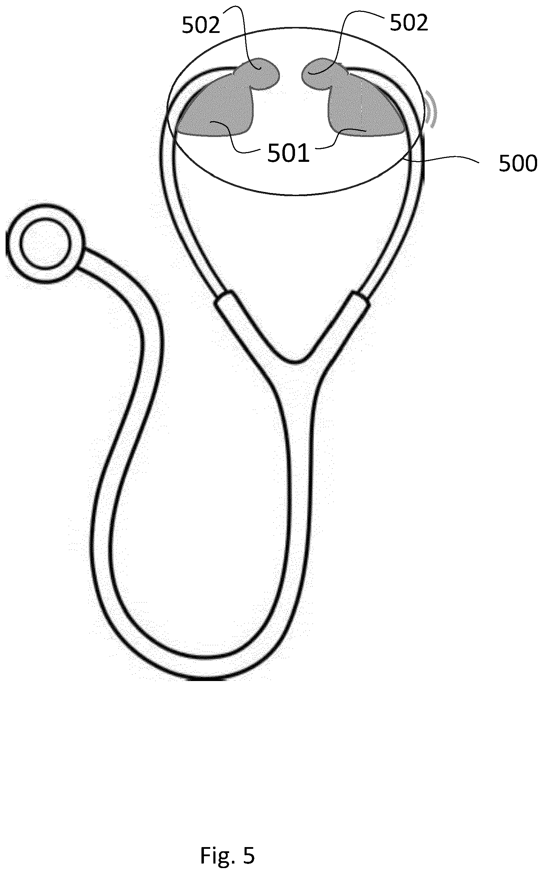

The figure 5 shows a fourth embodiment of the system 500 for digitizing the sound of a stethoscope 100, the stethoscope being equipped with eartips 101 and a tube, and for sending said sound to an electronic instrument comprising a body 501 having a processing function and that is integrated with a transducer-loudspeaker assembly 502, composed of a loudspeaker and a transducer, so that the entire system 500 is a single compact system acting as a substitute of the eartip 101 of the traditional stethoscope.

According to this fourth embodiment, in the system 500 the transducer is configured to digitize the sound coming from the stethoscope 100, the body 501 is configured to amplify, filter and send said sound to the loudspeaker, and the loudspeaker is configured to reproduce the sound processed by the body 501.

According to this fourth embodiment, also in the system 500 the transducer-loudspeaker assembly 502 is placed at one end of the stethoscope 100.

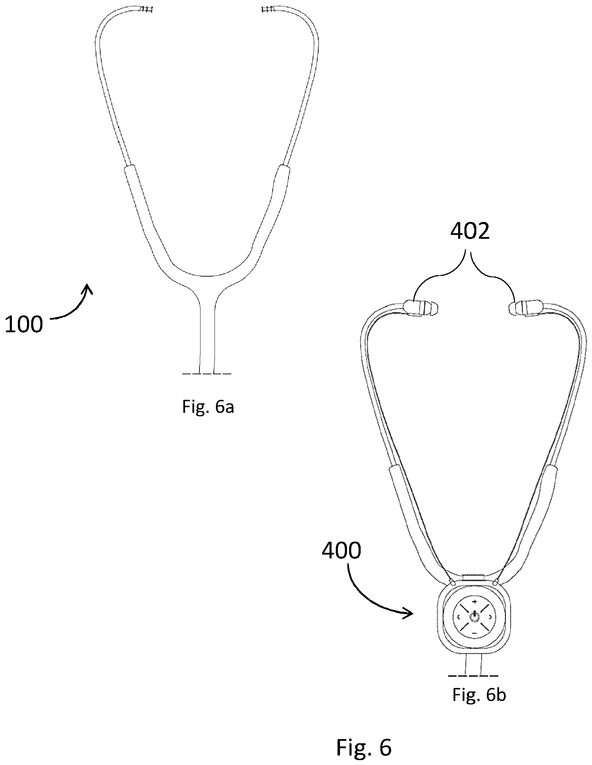

Advantageously according to the invention, the system 400 and 500 for digitizing the sound of a stethoscope and for sending it to an electronic instrument is configured to be applied to existing stethoscopes because the eartips of known stethoscopes are replaceable and interchangeable, as shown in figure 6.

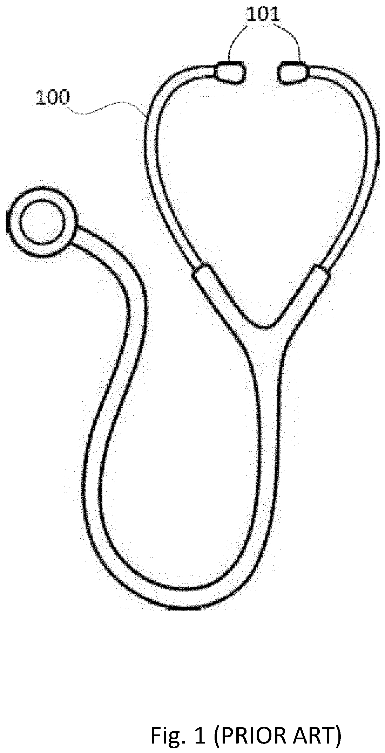

Figure 1 shows a known stethoscope, composed of a tube, replaceable eartips and a membrane. The eartips are positioned at the end of the tube to allow a user to position the stethoscope in his ears.

Figure 6a shows a known stethoscope without eartips.

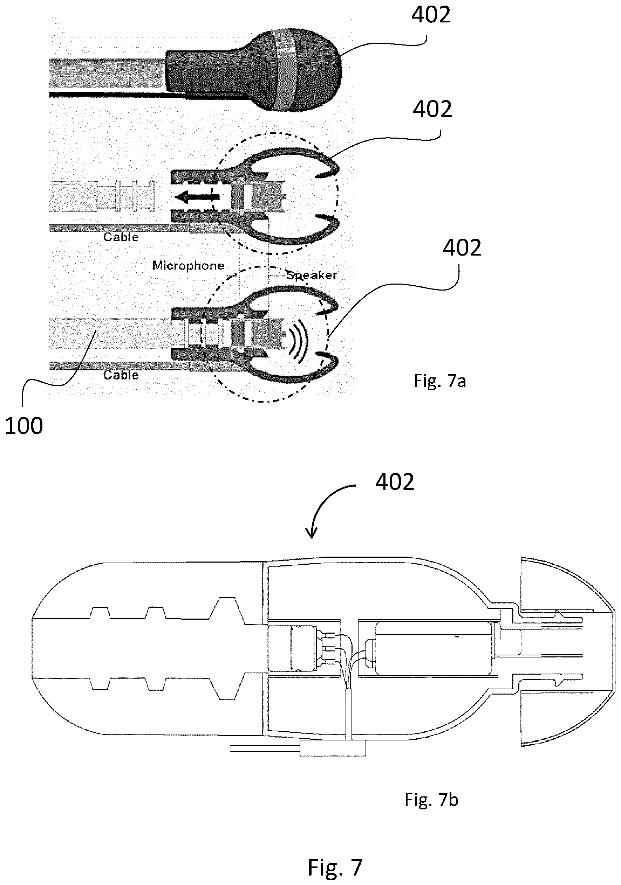

Figure 7 shows a transducer-loudspeaker assembly 202, 302, 402, 502 composed of a loudspeaker and a transducer, placeable at one end of the stethoscope 100.

Therefore, the system for digitizing the sound of a stethoscope and for sending it to an electronic instrument according to the invention allows to connect a traditional stethoscope to an electronic instrument, such as headphones or a smartphone or a tablet, possibly through a specific piece of software installed on these electronic devices.

In addition, the system to connect a stethoscope to a portable electronic device according to the invention is easily manufactured.

Another advantage of the system for digitizing the sound of a stethoscope and for sending it to an electronic instrument according to the invention is that it is easy.

Another advantage of the system for digitizing the sound of a stethoscope and for sending it to an electronic instrument according to the invention is that it is able to be applied to existing stethoscopes in a simple and practical way, without having to make changes.

Furthermore, the system for digitizing the sound of a stethoscope and for sending it to an electronic instrument according to the invention allows a remote monitoring of several diseases detectable only through an auscultation.

It is finally clear that the system for digitizing the sound of a stethoscope and for sending it to an electronic instrument here described and illustrated may be subject to modifications and variations without thereby departing from the scope of the present invention, as defined in the appended claims.

## Claims

1. System (400, 500) for digitizing the sound detected by a stethoscope (100) equipped with replaceable and interchangeable eartips (101) and a tube, and for sending the digitised sound to an electronic instrument, comprising:
- a transducer-loudspeaker assembly (402, 502) suitable for placement at one end of the stethoscope (100) and composed of a loudspeaker and a transducer, said transducer being configured to digitize the sound detected by of the stethoscope (100);
and
- a body (401, 501) configured to receive the digitized sound from the transducer and to amplify, filter and send it to the loudspeaker, said loudspeaker being configured to reproduce the sound processed by the body (401, 501) ;
**characterized in that** the transducer-loudspeaker assembly (402, 502) is suitable for direct connection to the tube of the stethoscope so as to turn into a substitute electronic eartip (101).

2. System (400, 500) according to claim 1, **characterized in that** the body ( 401) is configured to be hooked to the tube of the stethoscope (100) and is configured to receive via a cable or a wireless connection the digital signal from the transducer and to send the amplified and filtered digital signal via a cable or a wireless connection to the loudspeaker and to fixed or portable electronic devices.

3. System (400, 500) according to claim 1, **characterized in that** the body (401) is configured to be hooked to the tube of the stethoscope (100), and is configured to receive the digitized sound from the transducer and to amplify, filter and send it via a cable or a wireless connection to the transducer-loudspeaker assembly (402).

4. System (400, 500) according to claim 1, **characterized in that** the body (501) is integrated with the transducer-loudspeaker assembly (502) to substitute at least one of the eartips (101) and it is configured to receive the digitized sound from the transducer, and send the amplified and filtered sound to the loudspeaker is integrated with the transducer-loudspeaker assembly (502).

## Patentansprüche

1. System (400, 500) zum Digitalisieren des von einem Stethoskop (100) erfassten Schalls, das mit austauschbaren und austauschbaren Ohrstücken (101) und einem Schlauch ausgestattet ist, und zum Senden des digitalisierten Schalls an ein elektronisches Instrument, umfassend:
- Wandler-Lautsprecher-Anordnung (402, 502), die zur Platzierung an einem Ende des Stethoskops (100) geeignet ist und aus einem Lautsprecher und einem Wandler bestehteine , wobei der Wandler so konfiguriert ist, dass er den vom Stethoskop (100) erfassten Schall digitalisiert; und
- einen Körper (401, 501), der so konfiguriert ist, dass er den digitalisierten Ton vom Wandler empfängt und ihn verstärkt, filtert und an den Lautsprecher sendet, wobei der Lautsprecher so konfiguriert ist, dass er den von dem Körper (401, 501) verarbeiteten Ton wiedergibt;
**dadurch gekennzeichnet, dass** die Wandler-Lautsprecher-Baugruppe (402, 502) zum direkten Anschluss an den Schlauch des Stethoskops geeignet ist, so dass sie zu einem elektronischen Ersatz-Ohrstöpsel (101) wird.

2. System (400, 500) nach Anspruch 1, **dadurch gekennzeichnet, dass** der Körper (401) so konfiguriert ist, dass er mit dem Schlauch des Stethoskops (100) verbunden werden kann und so konfiguriert ist, dass er über ein Kabel oder eine drahtlose Verbindung das digitale Signal vom Wandler empfängt und das verstärkte und gefilterte digitale Signal über ein Kabel oder eine drahtlose Verbindung an den Lautsprecher und an stationäre oder tragbare elektronische Geräte sendet.

3. System (400, 500) nach Anspruch 1, **dadurch gekennzeichnet, dass** der Körper (401) so konfiguriert ist, dass er mit dem Schlauch des Stethoskops (100) verbunden werden kann, und so konfiguriert ist, dass er den digitalisierten Schall vom Wandler empfängt und ihn verstärkt, filtert und über ein Kabel oder eine drahtlose Verbindung an die Wandler-Lautsprecher-Anordnung (402) sendet.

4. System (400, 500) nach Anspruch 1, **dadurch gekennzeichnet, dass** der Körper (501) mit der Wandler-Lautsprecher-Baugruppe (502) integriert ist, um mindestens eine der Ohrstöpsel (101) zu ersetzen, und dass er so konfiguriert ist, dass er den digitalisierten Ton vom Wandler empfängt und den verstärkten und gefilterten Ton an den Lautsprecher sendet, der mit der Wandler-Lautsprecher-Baugruppe (502) integriert ist.

## Revendications

1. Système (400, 500) de numérisation du son détecté par un stéthoscope (100) équipé d'embouts remplaçables et interchangeables (101) et d'un tube, et d'envoi du son numérisé à un instrument électronique, comprenant :
- un ensemble transducteur-haut-parleur (402, 502) apte à être placé à une extrémité du stéthoscope (100) et composé d'un haut-parleur et d'un transducteur, ledit transducteur étant configuré pour numériser le son détecté par le stéthoscope (100) ; et
- un dispositif (401, 501) configuré pour recevoir le son numérisé du transducteur et pour l'amplifier, le filtrer et l'envoyer au haut-parleur, ledit haut-parleur étant configuré pour reproduire le son traité par le dispositif (401, 501) ;
**caractérisé par le fait que** l'ensemble transducteur-haut-parleur (402, 502) peut être raccordé directement au tube du stéthoscope pour devenir un embout électronique de remplacement (101).

2. Système (400, 500) selon la revendication 1, **caractérisé par le fait que** le dispositif (401) est configuré pour être accroché au tube du stéthoscope (100) et est configuré pour recevoir via un câble ou une connexion sans fil le signal numérique du transducteur et pour envoyer le signal numérique amplifié et filtré via un câble ou une connexion sans fil au haut-parleur et à des dispositifs électroniques fixes ou portables.

3. Système (400, 500) selon la revendication 1, **caractérisé en ce que** le dispositif (401) est configuré pour être accroché au tube du stéthoscope (100), et est configuré pour recevoir le son numérisé du transducteur et pour l'amplifier, le filtrer et l'envoyer via un câble ou une connexion sans fil à l'ensemble transducteur-haut-parleur (402).

4. Système (400, 500) selon la revendication 1, **caractérisé par le fait que** le dispositif (501) est intégré à l'ensemble transducteur-haut-parleur (502) pour remplacer au moins un des embouts (101) et qu'il est configuré pour recevoir le son numérisé du transducteur et pour envoyer le son amplifié et filtré au haut-parleur intégré à l'ensemble transducteur-haut-parleur (502).
